(19) Europäisches Patentamt
European Patent Office
Office européen des brevets

(11) EP 4 509 597 A1

(12) EUROPEAN PATENT APPLICATION
published in accordance with Art. 153(4) EPC

(43) Date of publication:
**19.02.2025 Bulletin 2025/08**

(21) Application number: **23788607.2**

(22) Date of filing: **12.04.2023**

(51) International Patent Classification (IPC):
***C12N 5/0775*** *(2010.01)* ***A61K 35/12*** *(2015.01)*
***A61P 29/00*** *(2006.01)* ***A61K 8/98*** *(2006.01)*

(52) Cooperative Patent Classification (CPC):
**A61K 8/98; A61K 35/12; A61K 35/28; A61P 29/00; A61Q 19/00; C12N 5/06**

(86) International application number:
**PCT/KR2023/004970**

(87) International publication number:
**WO 2023/200258 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2022 KR 20220045374**

(71) Applicants:
- **Inexoplat, Inc.**
  **Incheon 21984 (KR)**
- **Handong Global University Industry-Academic Cooperation Foundation**
  **Pohang-si, Gyeongsangbuk-do 37554 (KR)**

(72) Inventors:
- **KIM, Sung Hwan**
  **Incheon 21986 (KR)**
- **KIM, Yoon Young**
  **Incheon 22000 (KR)**
- **LEE, Jae Hwan**
  **Incheon 21986 (KR)**
- **YU, Ho Sung**
  **Incheon 22009 (KR)**
- **KO, Min Sung**
  **Incheon 21670 (KR)**
- **LEE, Hyeon Ji**
  **Incheon 21997 (KR)**
- **LEE, Jungmin**
  **Pohang-si, Gyeongsangbuk-do 37669 (KR)**
- **HONG, Eun Be**
  **Yongin-si, Gyeonggi-do 16874 (KR)**
- **SUH, Juhun**
  **Pohang-si, Gyeongsangbuk-do 37571 (KR)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

# (54) EXTRACELLULAR VESICLES ISOLATED FROM MESENCHYMAL STEM CELLS DERIVED FROM CHEMICALLY-INDUCED PLURIPOTENT STEM CELLS, AND USE THEREOF

(57) The present invention provides: a method for differentiating pluripotent stem cells into mesenchymal stem cells in the presence of a BMP4 signaling agonist; mesenchymal stem cells obtained thereby; and extracellular vesicles obtained from a culture solution of the stem cells. The extracellular vesicles have excellent anti-inflammatory effects, and thus can be used for treating inflammatory diseases. Therefore, there is a high possibility that a composition comprising the extracellular vesicles can be variously used as a pharmaceutical composition for treatment of inflammatory diseases and a cosmetic composition for skin improvement.

EP 4 509 597 A1

Figure 8

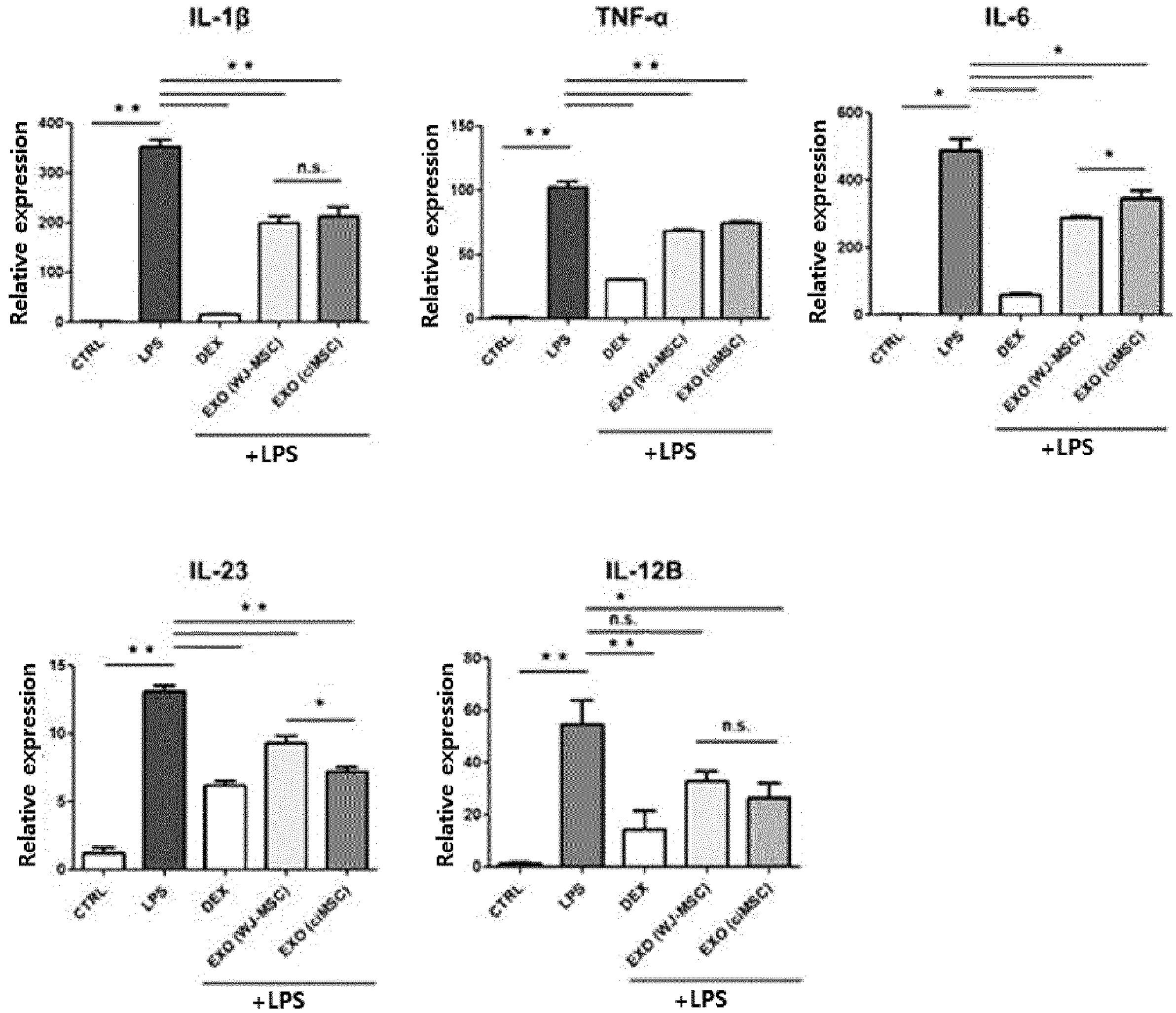
IL-1β
TNF-α
IL-6
IL-23
IL-12B
Relative expression
CTRL
LPS
DEX
EXO (WJ-MSC)
EXO (ciMSC)
+LPS
n.s.

## Description

### Technical Field

The present invention relates to a method for preparing mesenchymal stem cells from pluripotent stem cells using a compound, an extracellular vesicle obtained from a culture solution of the mesenchymal stem cells produced thereby, and a use thereof.

### Background Art

Pluripotent stem cells refer to stem cells with pluripotency that can differentiate into all three germ layers that make up the body, and thus can differentiate into all cells or organ tissues of the human body, and generally include embryonic stem cells. Human embryonic stem cells have many ethical issues because they are produced from embryos that can develop into human organisms, but they are known to have excellent cell proliferation and differentiation capabilities compared to adult stem cells.
Induced pluripotent stem cells (iPSCs) have been highlighted as an alternative cell source for regenerative medicine. Induced pluripotent stem cells can be obtained from multiple cells by combining compounds or genetic elements, and have high potential for use because they do not have ethical issues like embryonic stem cells. As a result, the discovery of human induced pluripotent stem cells has provided a new strategy in drug screening and therapeutic research for various diseases.
Mesenchymal stem cells are known to be involved in biological functions through regulating the microenvironment of damaged tissues, such as promoting angiogenesis, suppressing inflammation, and regulating immunity in the human body. These biological functions are not only the effects of the cells themselves, but also occur when various growth factors, cytokines, proteins that constitute the extracellular matrix, antioxidant proteins, and extracellular vesicles that promote the protection and regeneration of damaged tissues are secreted from mesenchymal stem cells. This is referred to as the paracrine effect.
Extracellular vesicles are vesicles composed of lipid bilayers, and comprise various types of substances such as proteins, lipids, and nucleic acids that exist inside the parent cell, and are known to play a very important role in the transmission of information between cells. In particular, extracellular vesicles isolated from mesenchymal stem cells are attracting attention as an alternative that can overcome various shortcomings of mesenchymal stem cell therapeutic agents because they have the therapeutic efficacy of mesenchymal stem cells while being cell-free.
Extracellular vesicles have the advantage of being safe because they do not induce immune responses and have a very low risk of tumorigenesis because they cannot self-replicate like cells. In addition, extracellular vesicles are much smaller than the cell size of 10,000 nm to 20,000 nm, so they can be administered without the risk of embolism, a potential risk factor for cell therapeutic agents. Currently, attempts are being made to apply the extracellular vesicles to dermatitis treatment (Korean Patent Application Publication No. 10-2019-0003336).
The therapeutic effects of extracellular vesicles isolated from mesenchymal stem cells vary greatly depending on the tissue from which the mesenchymal stem cells were extracted, the health condition of the donor, the culture method, and the like. Therefore, obtaining stem cells capable of producing extracellular vesicles with a certain therapeutic effect is very urgent.

### Detailed Description of Invention

### Technical Problem

Accordingly, the present inventors developed a method for preparing mesenchymal stem cells capable of effectively producing extracellular vesicles. In addition, the present inventors found that the extracellular vesicles isolated from mesenchymal stem cells have therapeutic efficacy similar to or superior to that of extracellular vesicles isolated from primary cultured mesenchymal stem cells. Based on the above, the present inventors completed the present invention.

### Solution to Problem

In order to achieve the above object, in one aspect of the present invention, there is provided a method for preparing mesenchymal stem cells, comprising: culturing pluripotent stem cells in a medium containing a BMP4 signaling agonist.
In another aspect of the present invention, there is provided a mesenchymal stem cell prepared by the above preparation method and an extracellular vesicle obtained from a culture solution thereof.
In another aspect of the present invention, there is provided a pharmaceutical composition for preventing or

treating an inflammatory disease, comprising the extracellular vesicle as an active ingredient.

## Effects of Invention

Mesenchymal stem cells (ciMSCs) can be differentiated from pluripotent stem cells by treatment with a BMP4 signaling agent. The mesenchymal stem cells (ciMSCs) thus differentiated exhibit general mesenchymal stem cell-specific marker expression. In addition, they possess all of the mesenchymal stem cell-specific traits of adipogenesis, chondrogenesis, and osteogenesis. Meanwhile, the extracellular vesicles isolated from the cells have the same physical properties as the extracellular vesicles isolated from primary mesenchymal stem cells, and it was confirmed that that their therapeutic efficacy is similar or superior. Therefore, the induced mesenchymal stem cells and the extracellular vesicles isolated therefrom prepared according to the present invention have therapeutic efficacy similar to or superior to primary mesenchymal stem cells and the extracellular vesicles isolated therefrom, respectively, and thus can be effectively utilized as a pharmaceutical composition or as an active ingredient for the treatment of various diseases.

## Brief Description of Drawings

Figure 1 shows a protocol for differentiating chemically induced mesenchymal stem cells (ciMSCs) from induced pluripotent stem cells (iPSCs).

Figure 2 shows the results obtained by confirming the characteristics of the mesenchymal stem cells (ciMSCs) prepared according to the present invention. Specifically, it shows the morphological characteristics of induced pluripotent stem cells (iPS cells), cells undergoing differentiation (Day 5), primary mesenchymal stem cells (BM-MSCs, WJ-MSCs), and ciMSCs. BM-MSC is bone marrow-derived MSC, WJ-MSC is Wharton's Jelly-derived MSC, and the scale bar is 100 μm.

Figure 3 shows the results of qRT-PCR analysis. The expression levels of OCT4, TBXT, SOX17, and NCAD genes in cells differentiating from induced pluripotent stem cells according to the present invention were compared. All data were normalized based on GAPDH and expressed as the mean ± SD. OCT4 is a pluripotent stem cell marker, TBXT is a mesoderm marker, SOX17 is a complete endoderm marker, and NCAD is a mesenchymal cell marker.

Figure 4 shows the results of flow cytometry analysis to confirm the expression of surface markers of the mesenchymal stem cells (ciMSCs) prepared according to the present invention. iPSC is an induced pluripotent stem cell, BM-MSC is a bone marrow-derived MSC, WJ-MSC is a Wharton's Jelly-derived MSC, and negative markers are CD34, CD11b, CD19, CD45, and HLA-DR.

Figure 5a shows the results obtained by confirming the differentiation potential of the mesenchymal stem cells (ciMSCs) prepared according to the present invention into adipocytes. BM-MSC is a bone marrow-derived MSC, WJ-MSC is a Wharton's Jelly-derived MSC, and the scale bar is 100 μm.

Figure 5b shows the results obtained by confirming the differentiation potential of the mesenchymal stem cells (ciMSCs) prepared according to the present invention into chondrocytes. BM-MSC is a bone marrow-derived MSC, WJ-MSC is a Wharton's Jelly-derived MSC, and the scale bar is 100 μm.

Figure 5c shows the results obtained by confirming the differentiation potential of the mesenchymal stem cells (ciMSCs) prepared according to the present invention into osteocytes. BM-MSC is a bone marrow-derived MSC, WJ-MSC is a Wharton's Jelly-derived MSC, and the scale bar is 100 μm.

Figure 6a shows the results obtained by confirming the cell number proliferation level (polulation doubling level, PDL) according to the culture period of the mesenchymal stem cells (ciMSCs) prepared according to the present invention and bone marrow-derived mesenchymal stem cells (BM-MSCs).

Figure 6b shows the results obtained by confirming the cell number proliferation level according to the passage culture of the mesenchymal stem cells (ciMSCs) prepared according to the present invention and bone marrow-derived mesenchymal stem cells (BM-MSCs).

Figure 7a shows the results obtained by measuring the size distribution of exosomes derived from the mesenchymal stem cells (ciMSCs) prepared according to the present invention and Wharton's jelly-derived mesenchymal stem cells (WJ-MSCs).

Figure 7b shows the results obtained by analyzing the surface protein expression of exosomes derived from the mesenchymal stem cells (ciMSCs) prepared according to the present invention and Wharton's jelly-derived mesenchymal stem cells (WJ-MSCs) by Western blot. CD63, CD81, CD9, and TSG101 are positive, and calnexin is negative.

Figure 8 shows the results obtained by confirming the anti-inflammatory effect of exosome derived from the mesenchymal stem cells (ciMSCs) prepared according to the present invention and Wharton's jelly-derived mesenchymal stem cells (WJ-MSCs). CTRL is control, DEX is dexamethasone, and LPS is lipopolysaccharide. All data

were normalized based on GAPDH. n = 3 to 5, *p < 0.05, **p < 0.01, ***p < 0.001. n.s. means not significant.

Figure 9 is an image showing a heat map of genes differentially expressed between iPSC, ci-MSC, BM-MSC, and WJ-MSC.

## Mode for Carrying out the Invention

### Method for preparing mesenchymal stem cells

In one aspect of the present invention, there is provided a method for preparing mesenchymal stem cells, comprising: culturing pluripotent stem cells in a medium containing a BMP4 signaling agonist (BMP4 agonist).

At this time, the BMP4 signaling agonist (BMP4 agonist) refers to a substance that induces BMP4 (bone morphogenetic protein 4) signaling. Preferably, the BMP4 signaling agonist may be SB4 (2-[[(4-bromophenyl)methyl]thio] benzoxazole). The SB4 may be a compound represented by the following formula 1:

[Formula 1]

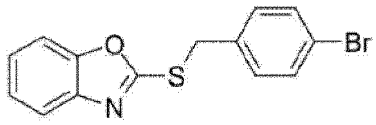

At this time, the medium may further comprise a TGF-β inhibitor, a ROCK inhibitor, a MEK inhibitor, a p38 MAPK inhibitor, a JNK inhibitor, a PKC inhibitor, and a GSK3 inhibitor. In addition, the culturing step may be performed for 2 to 8 days, and may also be performed for 2, 3, 4, 5, 6, 7, or 8 days.

The TGF-β inhibitor refers to a substance that inhibits TGF-β (Transforming Growth Factor-β). The TGF-β inhibitor may be any one selected from the group consisting of SB-431542, A83-01, LY-2109761, LY-2157299, RepSox, SB-505124, SB-525334, and a combination thereof. Preferably, the TGF-β inhibitor may be SB-431542.

The ROCK inhibitor refers to a substance that inhibits ROCK (Rho-associated protein kinase). The ROCK inhibitor may be any one selected from the group consisting of Y-27632, Y-39983, Thiazovivin, Fasudil, GSK429286A, RKI-1447, H-1152, Azaindole 1, and a combination thereof. Preferably, the ROCK inhibitor may be Y-27632.

The MEK inhibitor refers to a substance that targets MEK1/2 involved in the MEK (mitogen-activated protein kinase kinase) signaling process. The MEK inhibitor may be any one selected from the group consisting of PD0325901, PD184352 (CI-1040), U0126, U0126-EtOH, GSK1120212, PD98059, BIX 02189, SL-327, BIX 02188, AZD8330, TAK-733, PD318088, WX-554, GDC-0623, E6201, RO4987655, RO5126766, and a combination thereof. Preferably, the MEK inhibitor may be PD0325901.

The p38 MAPK inhibitor refers to a substance that inhibits p38 MAPK (mitogen-activated protein kinase). The p38 MAPK inhibitor may be any one selected from the group consisting of Pamapimod (Ro4402257), PH-797804, BIRB796, VX-702, SB239063, SCIO 469 hydrochloride, BMS-582949, Losmapimod (GW586553X), SB203580, SB202190, and a combination thereof. Preferably, the p38 MAPK inhibitor may be SB202190.

The JNK inhibitor refers to a substance that inhibits JNK (c-Jun N-terminal kinase). The JNK inhibitor may be any one selected from the group consisting of JNK Inhibitor V, JNK-IN-8, Resveratrol (SRT501), JNK inhibitor VIII, IQ3, DB07268, IQ-1S, Bentamapimod (AS602801), Tanzisertib (CC-930), SP600125, and a combination thereof. Preferably, the JNK inhibitor may be SP600125.

The PKC inhibitor refers to a substance that inhibits PKC (Protein kinase C). The PKC inhibitor may be any one selected from the group consisting of Enzastaurin (LY317615), GSK690693, Sotrastraurin (AEB071), Staurosporine (AM-2282), Bisindolylmaleimide I (GF109203X), Bisindolylmaleimide IX (Ro 31-8220 Mesylate), Ruboxistaurin (LY333531), Midostaurin (PKC412), Go6976, Go6983, and a combination thereof. Preferably, the PKC inhibitor may be Go6983.

The GSK3 inhibitor refers to a substance that inhibits GSK3 (Glycogen synthase kinase). The GSK3 inhibitor may be any one selected from the group consisting of SB216763, CHIR-98014, AZD1080, LY2090314, BRD0705, BIO-acetoxime, CHIR99021 (Laduviglusib), and a combination thereof. Preferably, the GSK inhibitor may be CHIR99021.

At this time, the medium may comprise 0.1 μM to 1 mM of a TGF-β inhibitor, 0.01 μM to 100 μM of a BMP4 signaling agonist, 0.05 μM to 500 μM of a ROCK inhibitor, 0.01 μM to 100 μM of a MEK inhibitor, 0.1 μM to 1 mM of a p38 MAPK inhibitor, 0.1 μM to 1 mM of a JNK inhibitor, 0.005 μM to 50 μM of a PKC inhibitor, and 0.3 μM to 3 mM of a GSK3 inhibitor.

In addition, the method for preparing mesenchymal stem cells may further comprise, after the above culturing, additionally culturing pluripotent stem cells in a medium containing a ROCK inhibitor, a MEK inhibitor, a p38 MAPK

inhibitor, a JNK inhibitor, a PKC inhibitor, and a GSK3 inhibitor.

At this time, the culturing step may be performed for 2 to 8 days, and may also be performed for 2, 3, 4, 5, 6, 7, or 8 days. In addition, the medium may comprise 0.05 μM to 500 μM of a ROCK inhibitor, 0.01 μM to 100 μM of a MEK inhibitor, 0.1 μM to 1 mM of a p38 MAPK inhibitor, 0.1 μM to 1 mM of a JNK inhibitor, 0.005 μM to 50 μM of a PKC inhibitor, and 0.3 μM to 3 mM of a GSK3 inhibitor.

At this time, the pluripotent stem cell may be an embryonic stem cell or an induced pluripotent stem cell.

As used herein, the term "mesenchymal stem cell (MSC)" refers to stem cells that exist in cartilage, bone tissue, adipose tissue, the stroma of bone marrow, and the like, differentiated from the mesoderm formed by division of a fertilized egg, and may include mesenchymal stem cells of animals, including mammals, for example humans. Mesenchymal stem cells maintain sternness and self-renewal and have the ability to differentiate into various cells, including chondrocytes, osteoblasts, muscle cells, and adipocytes, and can be extracted from bone marrow, adipose tissue, umbilical cord blood, synovial membrane, bone tissue (trabecular bone), muscle, infrapatellar fat pad, and the like. Mesenchymal stem cells have immunomodulatory abilities that suppress the activity and proliferation of T lymphocytes and B lymphocytes, inhibit the activity of natural killer cells (NK cells), and regulate the functions of dendritic cells and macrophages. In addition, mesenchymal stem cells have low immunogenicity and are therefore cells capable of allotransplantation and xenotransplantation.

**Mesenchymal stem cells**

In another aspect of the present invention, there is provided a mesenchymal stem cell prepared by the above preparation method. The mesenchymal stem cell may have the potential to differentiate into at least one cell selected from the group consisting of an adipocyte, a chondrocyte, and an osteocyte.

The mesenchymal stem cell may be positive for at least one marker selected from the group consisting of CD73, CD90, CD105, and CD166. The mesenchymal stem cell may be negative for at least one marker selected from the group consisting of CD11b, CD19, CD34, CD45, and HLA-DR.

Preferably, the mesenchymal stem cell i) may be positive for at least one marker selected from the group consisting of CD73, CD90, CD105, and CD166, and ii) may be negative for at least one marker selected from the group consisting of CD11b, CD19, CD34, CD45, and HLA-DR.

**Extracellular vesicles isolated from mesenchymal stem cells**

In another aspect of the present invention, there is provided an extracellular vesicle isolated from a culture solution of the prepared mesenchymal stem cells.

As used herein, the term "extracellular vesicle" refers to a nano-sized particle naturally secreted by living cells, packaged in a lipid bilayer, and may be a concept that includes all vesicles (exosomes, microvesicles, multivesicles, and extracellular vesicle-like vesicles) that serve as information transfer between cells and have a composition similar to that of extracellular vesicles.

As used herein, the term "isolated extracellular vesicle" refers to an extracellular vesicle substantially isolated from a cell from which an extracellular vesicle originates, for example, a mesenchymal stem cell.

The extracellular vesicle i) may be positive for at least one marker selected from the group consisting of CD9, CD63, CD81, and TSG101, and ii) may be negative for at least one marker selected from the group consisting of GM130 and calnexin.

As used herein, the term "positive" may mean, with respect to a specific marker, that the marker is present in a greater amount or at a higher concentration compared to a control. That is, the extracellular vesicle according to the present invention is positive for a marker when the marker is present on the surface or inside the extracellular vesicles and the extracellular vesicles can be distinguished from one or more non-extracellular vesicles or other extracellular vesicles using the marker. For example, the extracellular vesicle of the present invention can be detected with an antibody specific for CD81, an extracellular vesicle-specific surface antigen, and when the signal intensity from this antibody is detectably greater than that of the control (for example, background value), the extracellular vesicle is "CD81 positive ($CD81^+$)".

As used herein, the term "negative" means that the marker is not detectable compared to background values even when using a specific antibody. For example, when the extracellular vesicle of the present invention cannot be detectably labeled with an antibody specific for calnexin, the extracellular vesicle is "calnexin negative ($calnexin^-$)".

The above immunological characteristics can be determined by conventional methods known in the art to which the present invention pertains. For example, various methods such as flow cytometry, immunocytochemical staining, Western blot, or RT-PCR can be used.

### Use of extracellular vesicles

In another aspect of the present invention, there is provided a pharmaceutical composition for preventing or treating various diseases, comprising the extracellular vesicle as an active ingredient.

The disease may be any one selected from the group consisting of diseases selected from inflammatory diseases, diseases of the eye, diseases of the bone, neural diseases, neuronal diseases, neurodegenerative diseases, diseases of the skin, immune and/or autoimmune diseases, diseases of the mouth, metabolic diseases, cardiovascular diseases, proliferative diseases, diseases of the ear, diseases of the intestine, diseases of the respiratory system, and a combination thereof, and tumor (cancer), but is not limited thereto.

In one embodiment, the present invention provides a pharmaceutical composition for preventing or treating an inflammatory disease, comprising the extracellular vesicle as an active ingredient.

In addition, the present invention provides a use of the extracellular vesicle for the prevention or treatment of an inflammatory disease; or a use of the extracellular vesicle for the manufacture of a pharmaceutical composition for preventing or treating an inflammatory disease.

In addition, the present invention provides a method for preventing or treating an inflammatory disease, comprising administering the pharmaceutical composition to a subject.

The inflammatory disease may be any one selected from the group consisting of atopic dermatitis, allergic dermatitis, contact dermatitis, acne, seborrheic dermatitis, miliaria, urticaria, psoriasis, scleroderma, eczema, vitiligo, lupus, inflammatory bowel disease, Crohn's disease, nonalcoholic fatty liver disease, rheumatoid arthritis, graft versus host disease, Behcet's disease, seborrheic dermatitis, dementia, depression, and autism, but is not limited thereto.

The preferred dosage of the pharmaceutical composition varies depending on the patient's condition and body weight, the severity of the disease, the drug form, and the route and period of administration, but can be appropriately selected by those of ordinary skill in the art. In the pharmaceutical composition for preventing or treating an inflammatory disease of the present invention, the active ingredient may be included in any amount (effective amount) depending on the purpose, formulation, mixing purpose, and the like, as long as it can exhibit anti-inflammatory activity. Here, the "effective amount" refers to an amount of the active ingredient capable of inducing an anti-inflammatory effect. Such an effective amount can be experimentally determined within the normal capabilities of those of ordinary skill in the art.

The pharmaceutical composition may comprise the extracellular vesicles isolated from mesenchymal stem cells as an active ingredient in an amount of about 0.1 wt% to about 99 wt%, specifically about 0.5 wt% to about 90 wt%, and more specifically about 1 wt% to about 70 wt%, based on the total weight of the composition.

As used herein, the term "treatment" may be used to mean both therapeutic treatment and preventive treatment. At this time, prevention may be used to mean alleviating or reducing a pathological condition or disease in a subject. In one embodiment, the term "treatment" includes all applications or any form of medication for treating a disease in mammals, including humans. In addition, the above term includes inhibiting or slowing down a disease or the progression of a disease; restoring or repairing damaged or defective functions, thereby partially or completely alleviating a disease; or stimulating an inefficient process; or alleviating a serious disease.

Here, the term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined according to factors including the health condition of the patient, the type and severity of the disease, the activity of the drug, the sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective for treating an inflammatory disease.

The pharmaceutical composition may be administered as an individual therapeutic agent or in combination with another therapeutic agent, may be administered sequentially or simultaneously with a conventional therapeutic agent, and may be administered in one dose or in multiple doses. It is important to administer an amount that can achieve the maximum effect with the minimum amount without side effects by considering all of the above factors, and this can be easily determined by those of ordinary skill in the art.

The pharmaceutical composition may comprise conventional, non-toxic, pharmaceutically acceptable additives that are combined into a formulation according to a conventional method. For example, the pharmaceutical composition may further comprise a pharmaceutically acceptable carrier, diluent, or excipient.

The pharmaceutically acceptable carrier may be any carrier as long as it is non-toxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient

and does not have toxicity beyond what the application (prescription) target can adapt.

When the pharmaceutical composition is prepared as a parenteral formulation, it may be formulated in the form of an injection together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used. Methods for formulating pharmaceutical compositions are known in the art, and specific references can be made to literature [Remington's Pharmaceutical Sciences (19th ed., 1995)], etc. The above literature is considered a part of the present specification.

The administration method of the pharmaceutical composition is determined depending on the severity of skin inflammation symptoms, and topical administration is usually preferred. The administration may be performed directly to the affected area through an injection, and specifically, may be performed through subcutaneous injection.

The preferred dosage of the pharmaceutical composition may be in the range of 0.01 μg/kg to 10 g/kg per day, or in the range of 0.01 mg/kg to 1 g/kg per day, depending on the patient's condition, body weight, sex, and age, the severity of the patient, and the route of administration. The administration may be performed once a day or divided into several times per day. This dosage may be increased or decreased depending on the route of administration, the severity of the disease, gender, body weight, age, and the like, and therefore should not be construed as limiting the scope of the present invention in any way.

The subject to which the pharmaceutical composition can be applied (prescribed) is a mammal and a human, and particularly, the subject is preferably a human. In addition to the active ingredient, the pharmaceutical composition of the present invention may further comprise any compound or natural extract that has already been verified as safe and known to have therapeutic efficacy in order to increase or enhance the activity as a therapeutic agent.

The pharmaceutical composition may be applied to the skin. The formulation of the above pharmaceutical composition may be a formulation for skin external preparation. The above skin external preparation may be manufactured and used in the form of, for example, an ointment, a lotion, a spray, a patch, a cream, a powder, a suspension, a patch, or a gel, but is not particularly limited thereto.

In another aspect of the present invention, there is provided an anti-inflammatory cosmetic composition comprising the extracellular vesicle as an active ingredient. In addition, there is provided a use of the extracellular vesicle for the manufacture of a cosmetic composition.

The extracellular vesicle is the same as described above.

The cosmetic composition may be formulated into a cosmetic formulation commonly manufactured in the art, if necessary.

The cosmetic composition may be formulated into, for example, a solution, a suspension, an emulsion, a paste, a gel, a cream, a lotion, a powder, a soap, a surfactant-containing cleansing, an oil, a powder foundation, an emulsion foundation, a wax foundation, and a spray, but is not limited thereto. Specifically, it may be formulated as a flexible toner, a nourishing toner, a nourishing cream, a massage cream, an essence, an eye cream, a cleansing cream, a cleansing foam, a cleansing water, a pack, a spray, or a powder. In addition, when the formulation of the cosmetic composition is a paste, a cream, or a gel, it may comprise a carrier component selected from the group consisting of animal oil, vegetable oil, wax, paraffin, starch, tragacanth, cellulose derivatives, polyethylene glycol, silicone, bentonite, silica, talc, zinc oxide, and a mixture thereof.

In addition, when the formulation of the cosmetic composition is a solution or an emulsion, it may comprise a carrier component selected from the group consisting of a solvent, a solvating agent, an emulsifying agent, and a mixture thereof. Examples thereof may include water, ethanol, isopropanol, ethyl carbonate, ethyl acetate, benzyl alcohol, benzyl benzoate, propylene glycol, 1,3-butylglycol oil, glycerol aliphatic esters, polyethylene glycol, sorbitan fatty acid esters, and a mixture thereof.

In addition, when the formulation of the cosmetic composition is a suspension, it may comprise a liquid diluent such as water, ethanol, or propylene glycol, a suspending agent such as ethoxylated isostearyl alcohol, polyoxyethylene sorbitol ester, and polyoxyethylene sorbitan ester, and a carrier component selected from the group consisting of microcrystalline cellulose, aluminum metahydroxide, bentonite, agar, tragacanth, and a mixture thereof.

The carrier component may be included in an amount of about 1 to about 99.99 wt%, preferably about 80 to about 90 wt%, based on the total weight of the cosmetic composition.

**Method for preparing extracellular vesicles**

The extracellular vesicle may be prepared by a method comprising the following steps: a) culturing the mesenchymal stem cells; b) obtaining a culture solution during the culturing process; and c) filtering the obtained culture solution to remove particles larger than 200 nm and then isolating the extracellular vesicles.

The cell culture medium may be a serum-free culture medium. In addition, it may be a serum-free culture medium containing a specific additive to enhance a specific efficacy.

Duplicate contents are omitted in consideration of the complexity of the present specification, and terms not otherwise defined in the present specification have meanings commonly used in the art to which the present invention pertains.

Hereinafter, the present invention will be described in more detail through examples. These examples are intended only to illustrate the present invention, and it will be obvious to those of ordinary skill in the art that the scope of the present invention is not to be construed as being limited by these examples.

**Preparation Example 1. Culturing of induced pluripotent stem cells**

The induced pluripotent stem cells (iPSCs) (ACS-1019, ATCC) were cultured in E8 medium (05990, STEMCELL Technologies) supplemented with 10 ng/mL of bFGF (100-18B, Pepro Tech) on a vitronectin-coated plate. The induced pluripotent stem cells were isolated from the medium using 0.5 mM EDTA (E8008, Sigma).

**Example 1. Preparation of mesenchymal stem cells differentiated from induced pluripotent stem cells**

The induced pluripotent stem cells isolated in Preparation Example 1 were prepared in a 12-well plate coated with vitronectin, and cultured for 5 days in α-MEM medium supplemented with 10% fetal bovine serum (FBS) and 1% penicillin-streptomycin (PS) by adding a chemical substance cocktail (cocktail I) comprising SB4, SB431542, Y27632, PD0325901, SB202190, SP600125, Go6983, and CHIR99021. This is to induce differentiation into mesoderm. On the 5th day of culture, the cells isolated from the medium by treating with 0.25% Trypsin-EDTA were centrifuged at 300 RCF for 5 minutes.

Thereafter, a chemical substance cocktail (cocktail II) comprising Y27632, PD0325901, SB202190, SP600125, Go6983 and CHIR99021 was added to α-MEM medium supplemented with 10% FBS and 1% PS, and the collected cells were suspended, and then $1 \times 10^6$ cells per well were placed in a 6-well plate and cultured for 5 days to induce the differentiation into mesenchymal stem cells.

The differentiated cells (ciMSCs) were placed in a 6-well plate at 5 x $10^5$ cells and 3 x $10^5$ cells per well in that order, and cultured in α-MEM medium supplemented with 10% FBS and 1% PS or low-glucose DMEM medium supplemented with 10% FBS and 1% PS. Thereafter, $3 \times 10^5$ cells per well were placed in a 6-well plate and passaged. The information and concentration of the chemical substances used are as shown in Table 1 below. The overall process for inducing differentiation from iPSCs to MSCs is shown in Figure 1.

[Table 1]

| Chemical/Reagent | Working Concentration | Catalog number | Manufacturer |
|---|---|---|---|
| Lipopolysaccharide(LPS) | 10 ng/mL | L4391 | sigma |
| Dexamethasone(DEX) | 100 ng/mL | 11015 | Cayman |
| SB431542 | 10 μM | S4317 | sigma |
| SB4 | 1 μM | HY-124697 | MCE |
| Y27632 | 5 μM | 1293823 | biogems |
| PD0325901 | 1 μM | 3911091 | biogems |
| SB202190 | 10 μM | 1523072 | biogems |
| SP600125 | 10 μM | 1295666 | biogems |
| Go6983 | 0.5 μM | 1331975 | biogems |
| CHIR99021 | 3 μM | SML1046 | sigma |

**Comparative Example 1. Culturing of Wharton's jelly-derived mesenchymal stem cells (WJ-MSCs)**

The primary mesenchymal stem cells isolated from human Wharton's jelly (WJ-MSC) were cultured in α-MEM (Alpha Modification of Eagle's Minimum Essential Medium) medium supplemented with 10% fetal bovine serum (Corning, USA) and 1% penicillin-streptomycin on a 0.1% gelatin-coated cell plate. When the Wharton's jelly-derived mesenchymal stem cells reached 90% or more confluency, the cells were separated from the medium by treatment with 0.25% Trypsin-EDTA, and then centrifuged at 300 RCF for 5 minutes. The collected cells were suspended by adding fresh α-MEM medium supplemented with 10% fetal bovine serum (Corning, USA) and 1% penicillin-streptomycin and then passaged.

**Comparative Example 2. Culturing of bone marrow-derived mesenchymal stem cells (BM-MSCs)**

The primary mesenchymal stem cells isolated from bone marrow (BM-MSC) were cultured in $\alpha$-MEM (Alpha Modification of Eagle's Minimum Essential Medium) medium supplemented with 10% fetal bovine serum (Corning, USA) and 1% penicillin-streptomycin on a 0.1% gelatin-coated cell plate. When the bone marrow-derived mesenchymal stem cells reached 90% or more confluency, the cells were separated from the medium by treatment with 0.25% Trypsin-EDTA, and then centrifuged at 300 RCF for 5 minutes. The collected cells were suspended by adding fresh $\alpha$-MEM medium supplemented with 10% fetal bovine serum (Corning, USA) and 1% penicillin-streptomycin and then passaged.

**Experimental Example 1. Observation of morphological characteristics of ciMSC**

The morphological characteristics of iPS cells of Preparation Example 1, cells on the 5th day of differentiation induction in Example 1, differentiated ciMSC, WJ-MSC of Comparative Example 1, and BM-MSC of Comparative Example 2 were observed under a microscope and photographed, and the photographs are shown in Figure 2.

As shown in Figure 2, it was confirmed that the cells on the 5th day of differentiation induction had the morphological characteristics of both iPS cells and MSC, whereas the differentiated ciMSC had the morphological characteristics of mesenchymal stem cells derived from Wharton's jelly or bone marrow.

**Experimental Example 2. Measurement of expression level of specific cell-specific markers according to differentiation time**

The cells undergoing differentiation induction in Example 1 were collected according to differentiation time (12 hours, 24 hours, 48 hours, 72 hours), and induced pluripotent stem cells were used as a negative control. In order to confirm the expression level of cell-specific markers according to differentiation time, real-time quantitative polymerase chain reaction was performed.

Specifically, RNA was extracted and purified from the cells using TaKaRa MiniBEST universal RNA extraction kit (Takara Bio Inc., Japan). Complementary DNAs (cDNAs) were synthesized using the PrimeScript™ 1st strand cDNA synthesis kit (Takara Bio Inc., Japan). The expression levels of OCT4, TBXT, SOX17, and NCAD genes in cells undergoing differentiation were measured by performing RT-qPCR with the synthesized cDNA, and the results are shown in Figure 3. GAPDH was used as a reference gene for normalization of gene expression levels, and the sequence information of the primers used for RT-qPCR is as shown in Table 2 below.

[Table 2]

| Gene | Primer sequence | | SEQ ID NO | Annealing Temperature (°C) | Size (bp) |
|---|---|---|---|---|---|
| OCT4 | Forward | CCT GAA GCA GAA GAG GAT CAC C | 1 | 63.9 | 106 |
| | Reverse | AAA GCG GCA GAT GGT CGT TTG G | 2 | 63.9 | |
| TBXT | Forward | CCT TCA GCA AAG TCA AGC TCA CC | 3 | 62.2 | 153 |
| | Reverse | TGA ACT GGG TCT CAG GGA AGC A | 4 | 63.3 | |
| SOX17 | Forward | ACG CTT TCA TGG TGT GGG CTA AG | 5 | 63.5 | 112 |
| | Reverse | GTC AGC GCC TTC CAC GAC TTG | 6 | 64.1 | |
| NCAD | Forward | CCT CCA GAG TTT ACT GCC ATG AC | 7 | 64.9 | 149 |
| | Reverse | GTA GGA TCT CCG CCA CTG ATT C | 8 | 63.9 | |
| GAPDH | Forward | CAT CAC TGC CAC CCA GAA GAC TG | 9 | 63.3 | 153 |
| | Reverse | ATG CCA GTG AGC TTC CCG TTC AG | 10 | 65.2 | |

As shown in Figure 3, it was confirmed that as differentiation time elapses, the expression level of OCT4, a pluripotent cell-specific marker, is reduced; the expression level of TBXT, a mesoderm-specific marker, tends to increase; the expression level of NCAD, a marker specific for epithelial-mesenchymal transition (EMT), which is a process in which cells lose their epithelial properties and transform into highly mobile mesenchymal cells, is increased; and the expression level of SOX17, a definitive endoderm-specific marker, tends to decrease.

**Experimental Example 3. Confirmation of expression of mesenchymal stem cell-specific markers**

In order to confirm whether MSCs (ciMSCs) differentiated from induced pluripotent stem cells in Example 1 express mesenchymal stem cell-specific markers, flow cytometry was performed.

Specifically, $1 \times 10^5$ cells were collected from each of iPSCs of Preparation Example 1, WJ-MSCs of Comparative Example 1, BM-MSCs of Comparative Example 2, and ciMSCs of Example 1, centrifuged, and then suspended in 100 $\mu$L of PBS. For cell surface labeling, each cell was incubated with 0.25 to 0.5 $\mu$L of an MSC-specific surface marker antibody for 30 minutes, and then centrifuged again to be precipitated as a pellet. The precipitated cells were suspended in 200 $\mu$L of PBS, and then the expression of cell surface markers was analyzed by Attune NxT cytometer (Invitrogen), and the results are shown in Figure 4. CD90, CD 105, and CD73 were used as positive markers of mesenchymal stem cells, and CD34, CD11b, CD19, CD45, and HLA-DR were used as negative markers. The information of the antibodies used for flow cytometry is as shown in Table 3 below.

[Table 3]

| Target | Type | Producer | Dilution |
|---|---|---|---|
| Human CD90 | FITC (Clone : 5E10) | BD biosciences | 1:800 |
| Human CD105 | PerCP/Cy5.5 (Clone : 266) | BD biosciences | 1:800 |
| Human CD73 | PE/Cy7 (Clone : AD2) | BioLegend | 1:800 |
| Human CD34 | PE (Clone : 581) | BD biosciences | 1:800 |
| Human CD11b | PE (Clone : ICRF44) | BD biosciences | 1:800 |
| Human CD19 | PE (Clone : HIB19) | BD biosciences | 1:800 |
| Human CD45 | PE (Clone : HI30) | BD biosciences | 1:800 |
| Human HLA-DR | PE (Clone : G46-6) | BD biosciences | 1:800 |

As shown in Figure 4, 97.6% of the ciMSCs of Example 1 expressed CD73, 91.0% expressed CD90, 65.0% expressed CD105, and 6.3% expressed CD34, CD11b, CD19, CD45, or HLA-DR. These expression patterns were similar to those of WJ-MSCs and BM-MSCs and different from those of iPSCs, indicating that they maintained mesenchymal stem cell-specific characteristics.

**Experimental Example 4. Confirmation of differentiation potential** of **mesenchymal stem cells**

In order to confirm whether the ciMSCs of Example 1 had the ability of adipogenesis, chondrogenesis, and osteogenesis, the following experiment was performed. Specifically, for inducing differentiation into the tri-lineage, BM-MSCs of Comparative Example 2, WJ-MSCs of Comparative Example 1, and ciMSCs of Example 1 were placed on a 0.1% gelatin-coated plate (Adipogenesis: $5 \times 10^3/cm^2$, Chondrogenesis: $4.8 \times 10^4$/droplet, Adipogenesis: $1 \times 10^4/cm^2$), and differentiation was induced using a differentiation induction kit. During the differentiation period (Osteogenesis, Chondrogenesis: > 21 days, Adipogenesis: 7-14 days), the differentiation induction medium was replaced with fresh differentiation induction medium every 3 days.

The cells that had completed differentiation were washed with PBS and fixed with a 4% formaldehyde solution for 30 minutes. The fixed samples were stained with each staining reagent (Osteogenesis: Alizarin red, Chondrogenesis: Alcian blue, Adipogenesis: Oil Red O), and then observed under a microscope. The photographs taken are shown in Figures 5a to 5c. The differentiation induction kits and staining reagents including the differentiation induction medium used are as shown in Table 4 below.

[Table 4]

| Chemical/Reagent | Working Concentration | Catalog number | Manufacturer |
|---|---|---|---|
| Osteogenesis Differentiation Kit | 1X | A 10072-01 | gibco |
| Chondrogenesis Differentiation Kit | 1X | A 10071-01 | gibco |
| AdipogenesisDifferentiation Kit | 1X | A 10090-01 | gibco |
| Alizarin Red Solution | 1X | 2003999 | sigma |
| Alcian Blue Solution | 1% | TMS-010-C | sigma |

(continued)

| Chemical/Reagent | Working Concentration | Catalog number | Manufacturer |
|---|---|---|---|
| Oil Red O Solution | 0.3% | 01391 | sigma |
| Gentamicin | 5 μg/mL | G 1264 | sigma |

As shown in Figures 5a to 5c, it was confirmed that the ciMSCs of Example 1 have the characteristics of mesenchymal stem cells that can differentiate into adipocytes, chondrocytes, and osteocytes, similar to the WJ-MSCs of Comparative Example 1 and the BM-MSCs of Comparative Example 2.

**Experimental Example 5. Confirmation of proliferation ability according to culture period and passage culture of mesenchymal stem cells**

In order to confirm the long-term culture and proliferation ability of the ciMSCs of Example 1, PDL (population doubling level) according to culture period and passage culture was measured. Specifically, the ciMSCs of Example 1 and the BM-MSCs of Comparative Example 2 were each placed in a 12-well plate at $1 \times 10^5$ cells per well, and the total number of cells was calculated during passage culture. PDL was calculated using the following mathematical equation, and the results are shown in Figures 6a and 6b.

[Mathematical equation 1]

$$PDL = X + 3.322 (\log Y - \log Z).$$

X = initial population doubling level,
Y = number of cells initially added, and
Z = final number of cells at the end of the growth phase.

As shown in Figure 6a, it was confirmed that the number of cell divisions was reduced as the culture period of the BM-MSCs of Comparative Example 2 was increased, but the number of cell divisions of the ciMSCs of Example 1 continued to increase even after 30 days of culture, indicating that the cell proliferation ability was maintained. In addition, as shown in Figure 6b, it was confirmed that compared to the BM-MSCs of Comparative Example 2, the number of cell divisions of the ciMSCs of Example 1 was increased significantly as the number of passages was increased, indicating that the proliferation rate was fast. Through this, it was confirmed that the ciMSCs of Example 1 had proliferation ability that enabled long-term culture and multiple passages.

**Experimental Example 6. Comparison of gene expression between ciMSCs and mesenchymal stem cells**

In order to confirm the gene expression similarity between ciMSCs and primary MSCs (BM-MSCs, WJ-MSCs) and iPSCs, the results of gene expression profiling were visualized.

Specifically, RNA-sequencing was performed to confirm the differential expression of genes between ciMSCs and BM-MSCs. First, ciMSCs, WJ-MSCs, and BM-MSCs were placed in a 6-well plate at $2 \times 10^5$ cells per well and cultured for 5 days. Then, lysates were obtained by treating 2 wells of cells with Trizol. The obtained lysates were analyzed, and the genes were visualized as a heat map. At this time, Log2 (raw count + 0.001) normalization was applied, and z score normalization was applied to the Row-scale.

As a result, as shown in Figure 9, the gene expression patterns of ciMSCs and other MSCs were compared, and it was confirmed that ciMSCs had similar gene expression profiling, especially with WJ-MSCs.

Specifically, genes differentially expressed in iPSCs compared to other MSCs (Category 1: 106 genes), genes differentially expressed in BM-MSCs compared to other MSCs (Category 2: 99 genes), genes differentially expressed in ciMSCs compared to other MSCs (Category 3: 19 genes), and genes differentially expressed in WJ-MSCs compared to other MSCs (Category 4: 3 genes) were distinguished.

As shown in Figure 9, genes down-expressed in iPSCs compared to iPSCs and other MSCs (WJ-MSCs and BM-MSCs) are as follows:

AC112229.3, GGT1, ABCA12, PTHLH, CD274, CD226, OXTR, RRAD, FILIP1L, ST3GAL5, PSORS1C1, SCD5, TCF21, WNK4, IER3, TXNIP, SPRN, PCDH9, LYPD1, CA12, GREM2, FBLN7, AC114744.1, GPR68, AC022034.1, SGIP1, NOTCH2NLB, STAT4, AL354740.1, MYOZ2, HRCT1, MMP1, LINC01614, NLRP10, MGP, C1QTNF5, AL049629.2, LINC01204, IFI44, PLCE1-AS1, LNCOG, LINC00565, RGS4, DCN, VCAM1, FAP, PIGZ, LINC02454, AC007750.1, OSR1, GALNT5, TACR2, MFAP5, PHEX, PODN, LYST, CCDC190, LUM, MIRLET7BHG, CFI, PTGDS, TGFBR2, CSF1,

THBS1, EHBP1L1, AHNAK2, IGFBP6, TNFAIP6, JCAD, PLAT, RAPH1, DPP7, HAPLN1, PMP22, LGALS3, OSGIN1, NOTCH2NLA, ENG IGIP, VDR, AC245297.1, SPHK1, AC037198.2, IFI6, CMAHP, CTSB, LHFPL6, TM4SF1, NTNG2, TFPI, TBC1D2, IGFBP4, LINC01119, SIGLEC15, AHR, EVI2A, PAG1, AC004967.1, CYP4V2, MALINC1, STS, CRIP2, NTN4, FMN2, CPQ, AC020763.3.

As shown in Figure 9, genes down-expressed in BM-MSCs compared to iPSCs and other MSCs (WJ-MSCs and ciMSCs) are as follows:

DSC2, DHRS2, AOC3, STAR, AC022415.3, FCHO1, C1orf226, DLGAP1, TESMIN, AFDN-DT, IGSF11, ITGA2B, LINC00173, AC074135.1,, OTULINL" SCN5A,, COL4A6,, FGFR4,, CXADR, TPTE2P1, EFNA3, RUNDC3A, SETSIP, AF106564.1, PRKCQ-AS1, CADM3, AC093866.1, HS3ST5, NSG1, NODAL, VWDE, AC020928.1, N4BP3, LINGO1, CTNNA2, PDE3B, BLACAT1, GRB7, KRTCAP3, GUCA1A, CBX2, GPC2, STMN1, PTPRD, GCA, SALL2, AC068587.4, PPP2R2B, BLM, SINHCAF, TMEM92, PMAIP1, KLHL23, LLGL2, NUDT10, GNAO1, MBOAT1, COL9A3, TET1, SEMA6B, CA14, EPCAM, CGN, RCOR2, NEURL1B, F11R, UBE2C, MYBL2, BUB1B, DLGAP5, SPC25, PIMREG, KIF20A, NUSAP1, TROAP, COL26A1, PRKCZ, SOX15, DRAXIN, SMPDL3B, GRIP1, RNF43, CDCA7, EPHX2, PRXL2A, IQGAP2, PRKAR2B, WDR17, CERS4, SKA1, CHST6, RND2, MAP2K6, GABRB3, LMNB1, RPS6KA1, RAB11FIP4, CDK18, TMPO-AS1.

As shown in Figure 9, genes down-expressed in ciMSCs compared to iPSCs and other MSCs (WJ-MSCs and BM-MSCs) are as follows:

MST1R, KCNE3, KLHDC9, NUDT16P1, AC011447.6, PDE2A, RAB37, AC239809.3, AC098582.1, FBP1, AC138811.2, ALOX12P2, APELA, IPOSP1, METTL7A, ALPL, GREB1, FAM124A, HTR7.

As shown in Figure 9, genes down-expressed in WJ-MSCs compared to iPSCs and other MSCs (ciMSCs and BM-MSCs) are as follows: CDH3, RIMS4, SELENOP.

**Example 2. Isolation of exosomes from ciMSCs**

The ciMSCs of Example 1 were cultured, and then the culture solution was obtained, and centrifuged at 2,000 RCF for 10 minutes at 4 °C. The supernatant of the separated solution was obtained, and particles larger than 200 nm were removed by filtration with 0.2 μm Minisart™ NML String Filters (Sartorious, Germany). Thereafter, exosomes were isolated by centrifugal forced filtration method using a Labspinner (Live Cell Instrument, South Korea). The isolated exosomes were washed twice with PBS and used immediately or stored at -20°C.

**Comparative Example 3. Isolation of exosomes from WJ-MSCs**

The exosomes were isolated in the same manner as described in Example 2, using the WJ-MSCs of Comparative Example 1 instead of the ciMSCs of Example 1, and stored.

**Experimental Example 7. Analysis of characteristics of exosomes**

**Experimental Example 7.1. Measurement of exosome size**

The size and size-specific particle distribution of the exosomes isolated from the ciMSCs of Example 2 and the exosomes isolated from the WJ-MSCs of Comparative Example 3 were compared. Specifically, in order to quantify the exosomes using NanoSight NS300 (Malvern Panalytical), 20 μL of exosome sample was diluted with 980 μL of PBS and loaded for analysis. PBS was used as a background control, and the number of exosomes was measured by subtracting the PBS measurement value from the sample measurement value. The analysis of each sample was performed in triplicate, and the results are shown in Figure 7a.

As shown in Figure 7a, the exosomes isolated from the WJ-MSCs of Comparative Example 3 and the exosomes isolated from the ciMSCs of Example 2 were mainly distributed in the size of 50 nm to 200 nm. Through this, it was confirmed that they were extracellular vesicles, particularly vesicles with the physical characteristics of exosomes.

**Experimental Example 7.2. Confirmation of marker expression of exosomes**

In order to confirm whether the exosomes isolated from the ciMSCs of Example 2 (ciMSC exo) and the exosomes isolated from the WJ-MSCs of Comparative Example 3 (WJ-MSC exo) express exosome-specific markers, Western blot was performed.

Specifically, RIPA (Radio Immunoprecipitation Assay) buffer was added to the exosome samples and vortexed for 5 seconds. The samples were incubated at room temperature for 5 minutes, and then Laemmli buffer was added and boiled at 95 °C for 5 minutes. The samples were loaded onto a 10% polyacrylamide gel and run at 140 V for 75 minutes. The samples on gel were transferred to PVDF membranes by a wet/tank blotting system (1703930, Bio-Rad) at 80 V, 200 mA

for 75 minutes.

The membranes were then washed 5 times with 1X TBST and blocked with 5% skim milk for 1 hour. Thereafter, the membrane was reacted with primary antibodies overnight at 4 °C. CD63, CD81, CD9, and TSG101 were used as positive markers of exosomes, and Calnexin was used as a negative marker of exosomes. After five washing steps with 1X TBST, it was reacted with a secondary antibody for 2 hours at room temperature. The membrane was treated with ECL (enhanced chemiluminescence) substrate (32106, Thermo Fisher scientific) or Femto substrate (34095, Thermo Fisher scientific). The information of the antibodies used is as shown in Table 5 below, and the results of Western blot are shown in Figure 7b.

[Table 5]

| Target | Type | Producer | Dilution |
|---|---|---|---|
| Human CD63 | mouse IgG (primary) | abcam | 1:500 |
| Human CD81 | mouse IgG (primary) | Bioss | 1:200 |
| Human CD9 | rabbit IgG (primary) | Bioss | 1:200 |
| Human Calnexin | rabbit IgG (primary) | Bioss | 1:200 |
| Human TSG101 | mouse IgG (primary) | abcam | 1:200 |
| Mouse IgG | goat IgG-HRP (secondary) | abcam | 1:10000 |
| Rabbit IgG | goat IgG-HRP (secondary) | abcam | 1:10000 |

As shown in Figure 7b, both the exosomes isolated from the WJ-MSCs of Comparative Example 3 (WJ-MSC exo) and the exosomes isolated from the ciMSCs of Example 2 (ciMSC-exo) expressed CD9, CD63, and CD81, which are membrane proteins of exosomes and belong to the Tetraspanin family, and TSG101, a multivesicular body protein, but did not express Calnexin, an integral membrane protein present in the endoplasmic reticulum. Through this, it was confirmed that they had the characteristics of exosomes.

**Experimental Example 8. Confirmation of *in vitro* anti-inflammatory effect of exosomes**

RAW 264.7 cells, which are mouse macrophagic cell line, were placed in a 48-well plate at $1 \times 10^5$ cells and cultured for 24 hours, and then the cells were treated with serum-free high-glucose DMEM medium containing lipopolysaccharide (LPS) to induce an inflammatory response. Thereafter, the cells were treated with dexamethasone (DEX), which suppresses the inflammatory response, the exosomes isolated from the WJ-MSCs of Comparative Example 3 (WJ-MSC EXO), or the exosomes isolated from the ciMSCs of Example 2 (ciMSC EXO), respectively, and cultured for 4 hours. After the culture was completed, RNA was extracted from RAW 264.7 cells in the same manner as described in Experimental Example 2, and analyzed by RT-qPCR. The results are shown in Figure 8, and the sequence information of the primers used is as shown in Table 6 below.

[Table 6]

| Gene | Primer sequence | | SEQ ID NO | Annealing Temperature (°C) | Size (bp) |
|---|---|---|---|---|---|
| IL-1β | Forward | GTT CCC ATT AGA CAA CTG CAC TAC AG | 11 | 66.3 | 162 |
| | Reverse | GTC GTT GCT TGG TTC TCC TTG TA | 12 | 62.9 | |
| TNF-α | Forward | ACG GCA TGG ATC TCA AAG AC | 13 | 60.1 | 116 |
| | Reverse | GTG GGT GAG GAG CAC GTA GT | 14 | 60.2 | |
| IL-6 | Forward | GAG GAT ACC ACT CCC AAC AGA CC | 15 | 66.4 | 141 |
| | Reverse | AAG TGC ATC ATC GTT GTT CAT ACA | 16 | 60.1 | |
| IL-23 | Forward | TGC GTG AAG GGC AAG GAC AC | 17 | 62.5 | 113 |
| | Reverse | AGG CCAAGG GCT CGA GAC TT | 18 | 62.5 | |
| IL-12B | Forward | AGG TCA CAC TGG ACC AAA GG | 19 | 60.5 | 173 |
| | Reverse | TGG TTT GAT GAT GTC CCT AG | 20 | 56.4 | |

As shown in Figure 8, when treated with the exosomes isolated from the ciMSCs of Example 2 (ciMSC EXO) after inducing an inflammatory response, it was confirmed that the expression levels of inflammatory cytokines IL-1β, TNF-α, IL-6, IL-23, and IL-12B, which had significantly increased due to the inflammatory response, were significantly reduced. Through this, it was confirmed that the anti-inflammatory effect of the exosomes isolated from ciMSCs (ciMSC EXO) is excellent.

## Claims

1. A method for preparing mesenchymal stem cells, comprising: culturing pluripotent stem cells in a medium comprising a BMP4 signaling agonist (BMP4 agonist).

2. The method for preparing mesenchymal stem cells according to claim 1, wherein the BMP4 signaling agonist is a compound represented by the following formula 1:

[Formula 1]

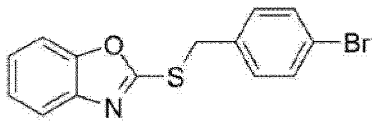

3. The method for preparing mesenchymal stem cells according to claim 1, wherein the medium further comprises a TGF-β inhibitor, a ROCK inhibitor, a MEK inhibitor, a p38 MAPK inhibitor, a JNK inhibitor, a PKC inhibitor, and a GSK3 inhibitor.

4. The method for preparing mesenchymal stem cells according to claim 1, wherein method further comprises, after the above culturing, additionally culturing pluripotent stem cells in a medium containing a ROCK inhibitor, a MEK inhibitor, a p38 MAPK inhibitor, a JNK inhibitor, a PKC inhibitor, and a GSK3 inhibitor.

5. The method for preparing mesenchymal stem cells according to claim 1, wherein the pluripotent stem cell is an embryonic stem cell or an induced pluripotent stem cell.

6. A mesenchymal stem cell prepared by the method according to claim 1.

7. The mesenchymal stem cell according to claim 6, wherein the mesenchymal stem cell is

    i) positive for at least one marker selected from the group consisting of CD73, CD90, CD105, and CD166, and
    ii) negative for at least one marker selected from the group consisting of CD 11b, CD19, CD34, CD45, and HLA-DR.

8. An extracellular vesicle isolated from a culture solution of the mesenchymal stem cells according to claim 6.

9. A pharmaceutical composition for preventing or treating an inflammatory disease, comprising the extracellular vesicle according to claim 8 as an active ingredient.

10. The pharmaceutical composition according to claim 9, wherein the inflammatory disease is any one selected from the group consisting of atopic dermatitis, allergic dermatitis, contact dermatitis, acne, seborrheic dermatitis, miliaria, urticaria, psoriasis, scleroderma, eczema, vitiligo, lupus, inflammatory bowel disease, Crohn's disease, nonalcoholic fatty liver disease, rheumatoid arthritis, graft versus host disease, Behcet's disease, seborrheic dermatitis, dementia, depression, and autism.

11. An anti-inflammatory cosmetic composition, comprising the extracellular vesicle according to claim 8 as an active ingredient.

12. A method for preventing or treating an inflammatory disease, comprising administering the extracellular vesicle according to claim 8 to a subject.

**13.** A use of the extracellular vesicle according to claim 8 for the prevention or treatment of an inflammatory disease.

Figure 1

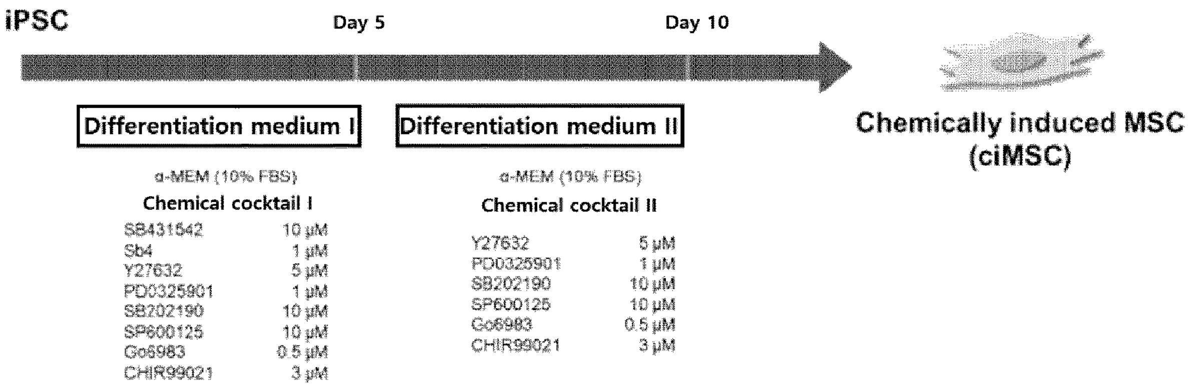


Figure 2

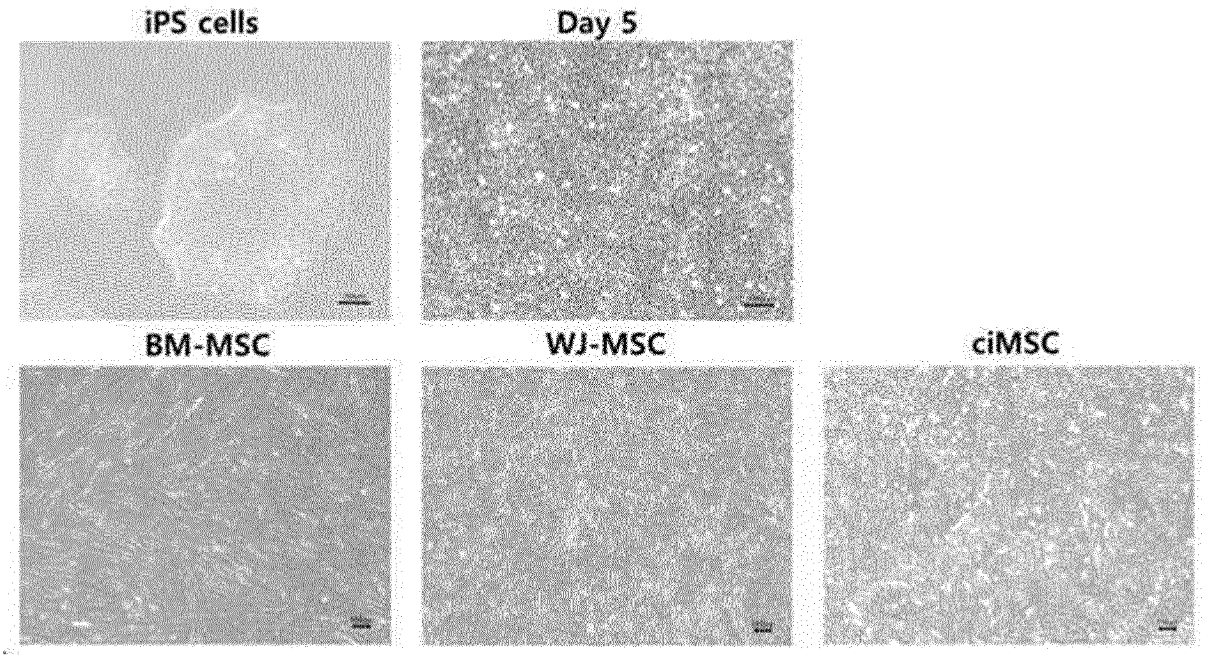

Figure 3

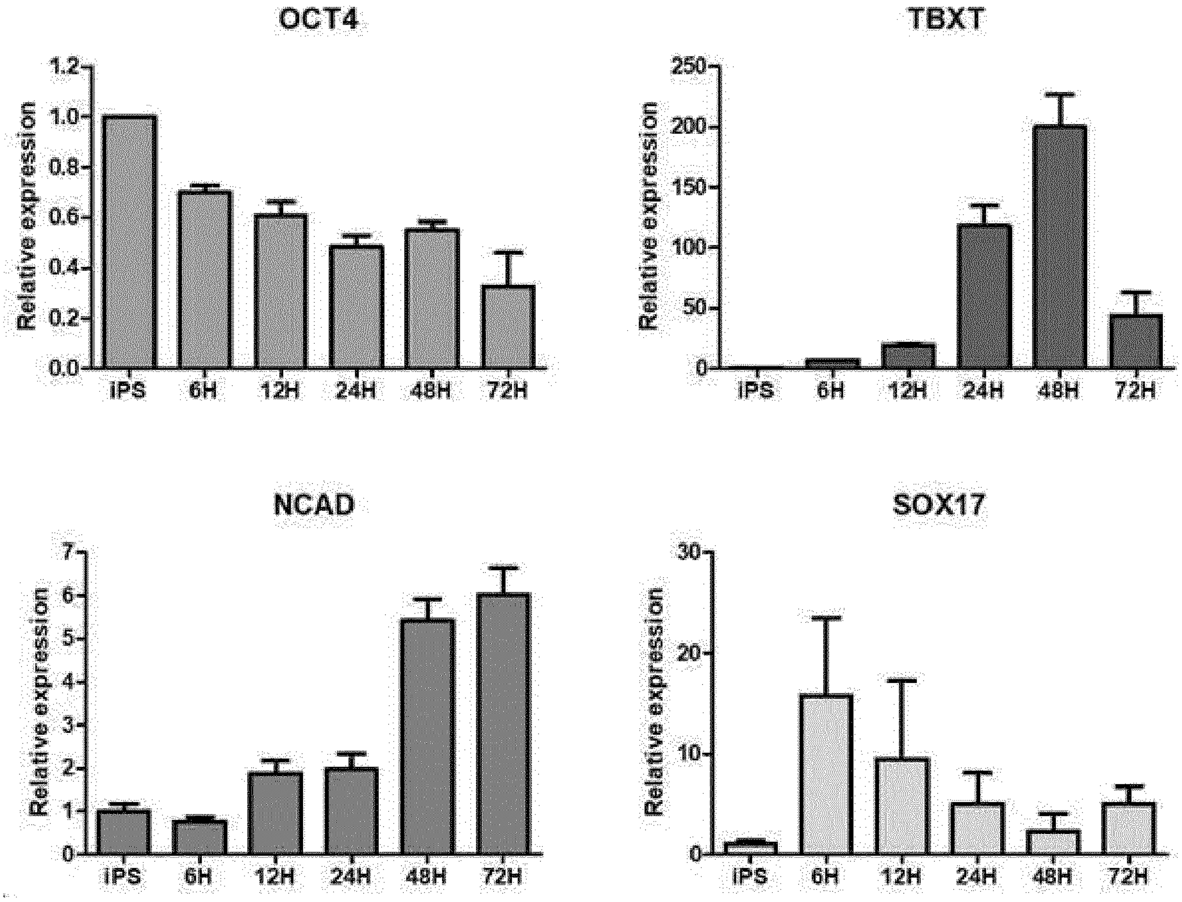
OCT4
Relative expression
1.2
1.0
0.8
0.6
0.4
0.2
0.0
iPS
6H
12H
24H
48H
72H
TBXT
Relative expression
250
200
150
100
50
0
iPS
6H
12H
24H
48H
72H
NCAD
Relative expression
7
6
5
4
3
2
1
0
iPS
6H
12H
24H
48H
72H
SOX17
Relative expression
30
20
10
0
iPS
6H
12H
24H
48H
72H

Figure 4

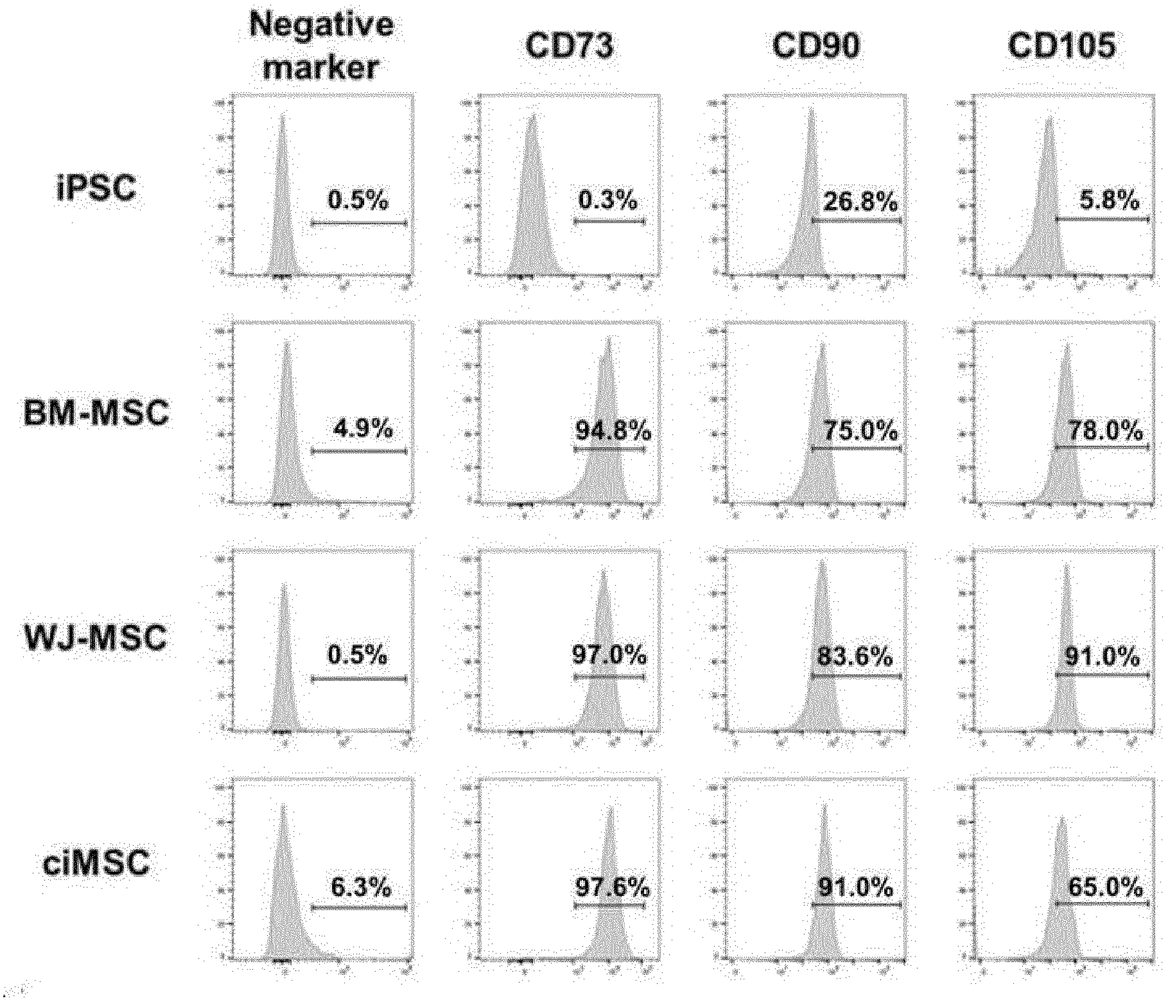
Negative marker
CD73
CD90
CD105
iPSC
0.5%
0.3%
26.8%
5.8%
BM-MSC
4.9%
94.8%
75.0%
78.0%
WJ-MSC
0.5%
97.0%
83.6%
91.0%
ciMSC
6.3%
97.6%
91.0%
65.0%

Figure 5a

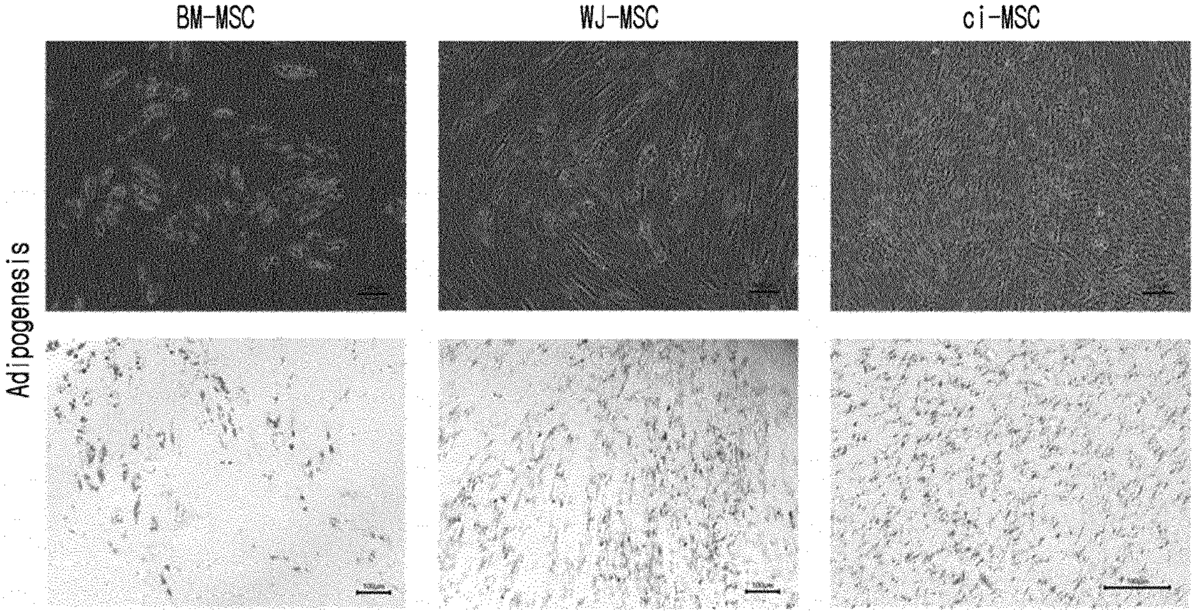


Figure 5b

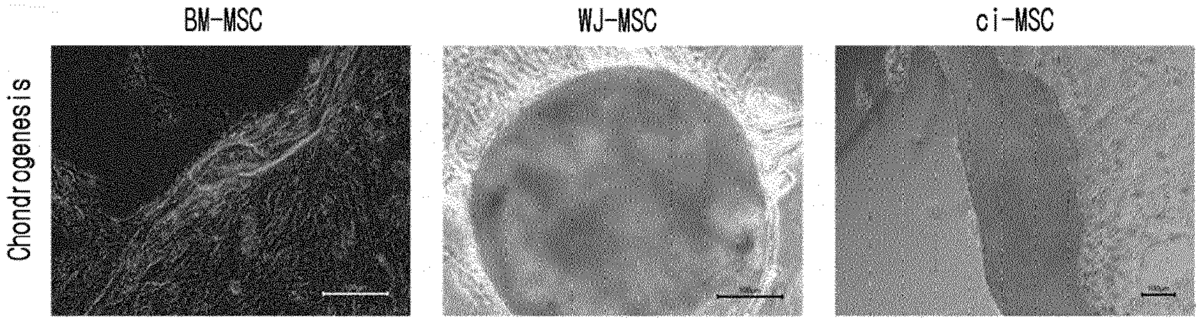


Figure 5c

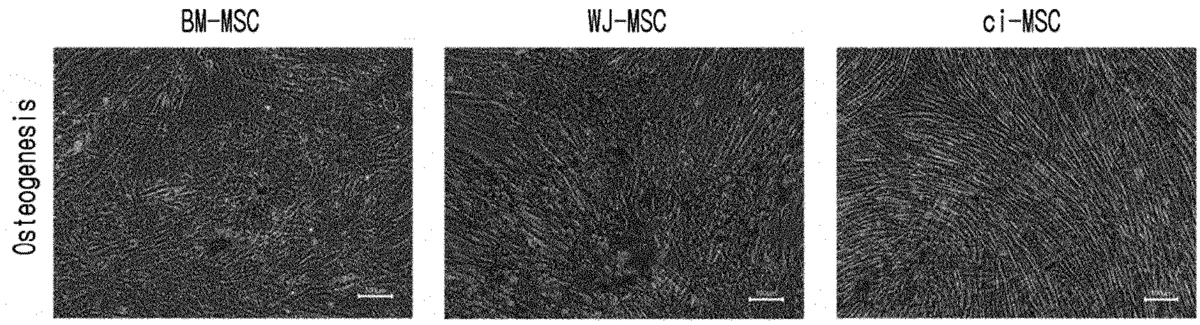

Figure 6a

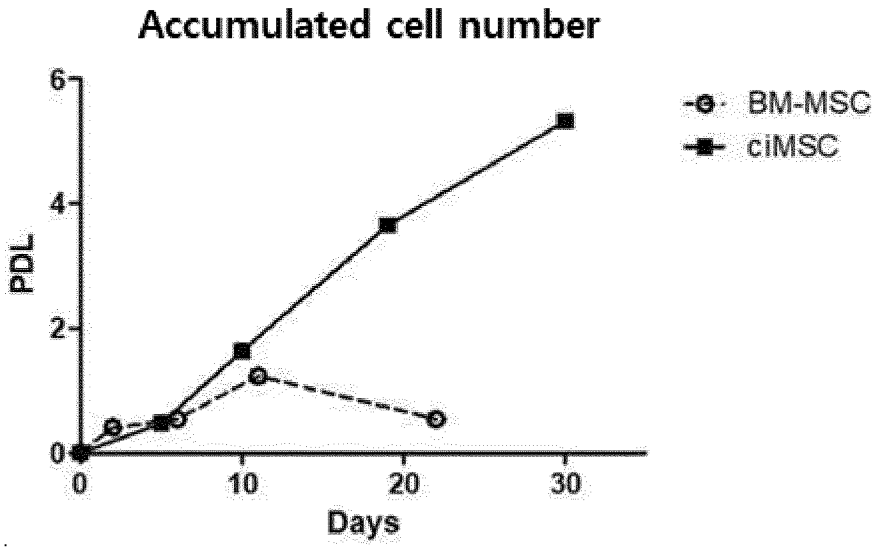


Figure 6b

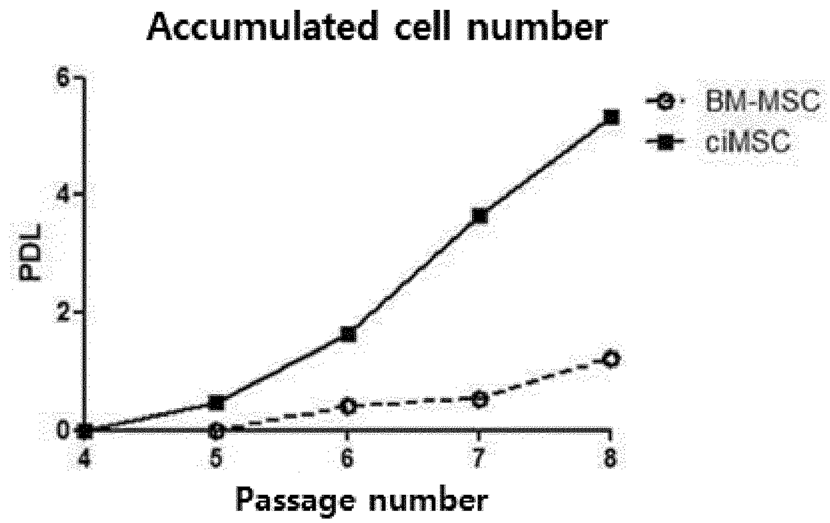

Figure 7a

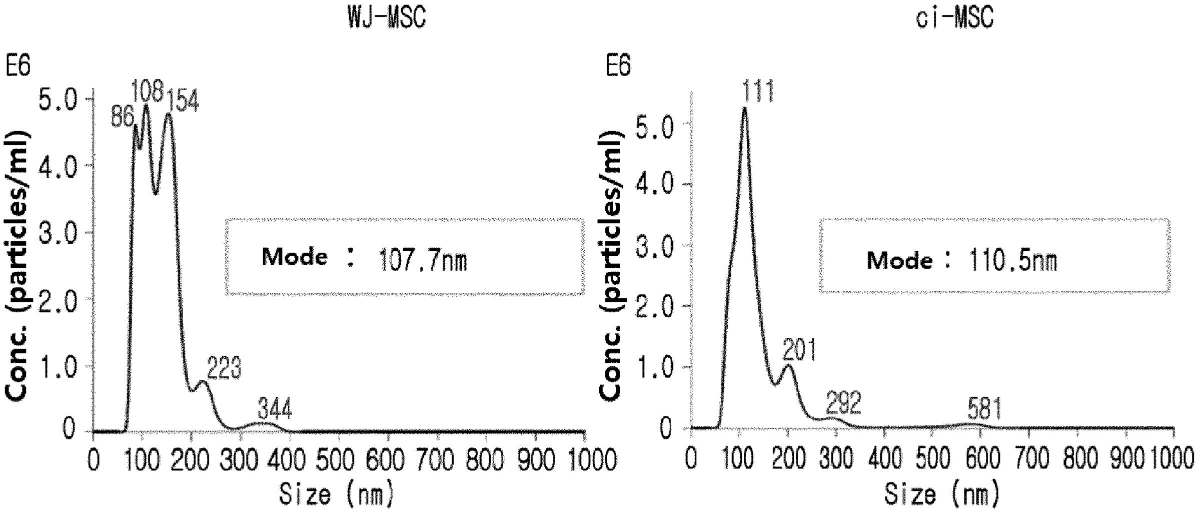


Figure 7b

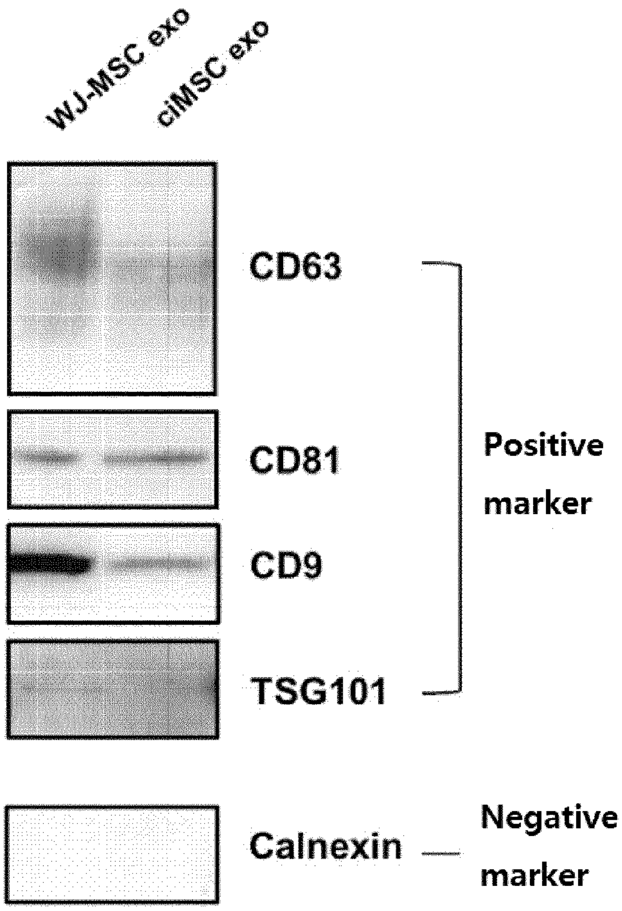

Figure 8

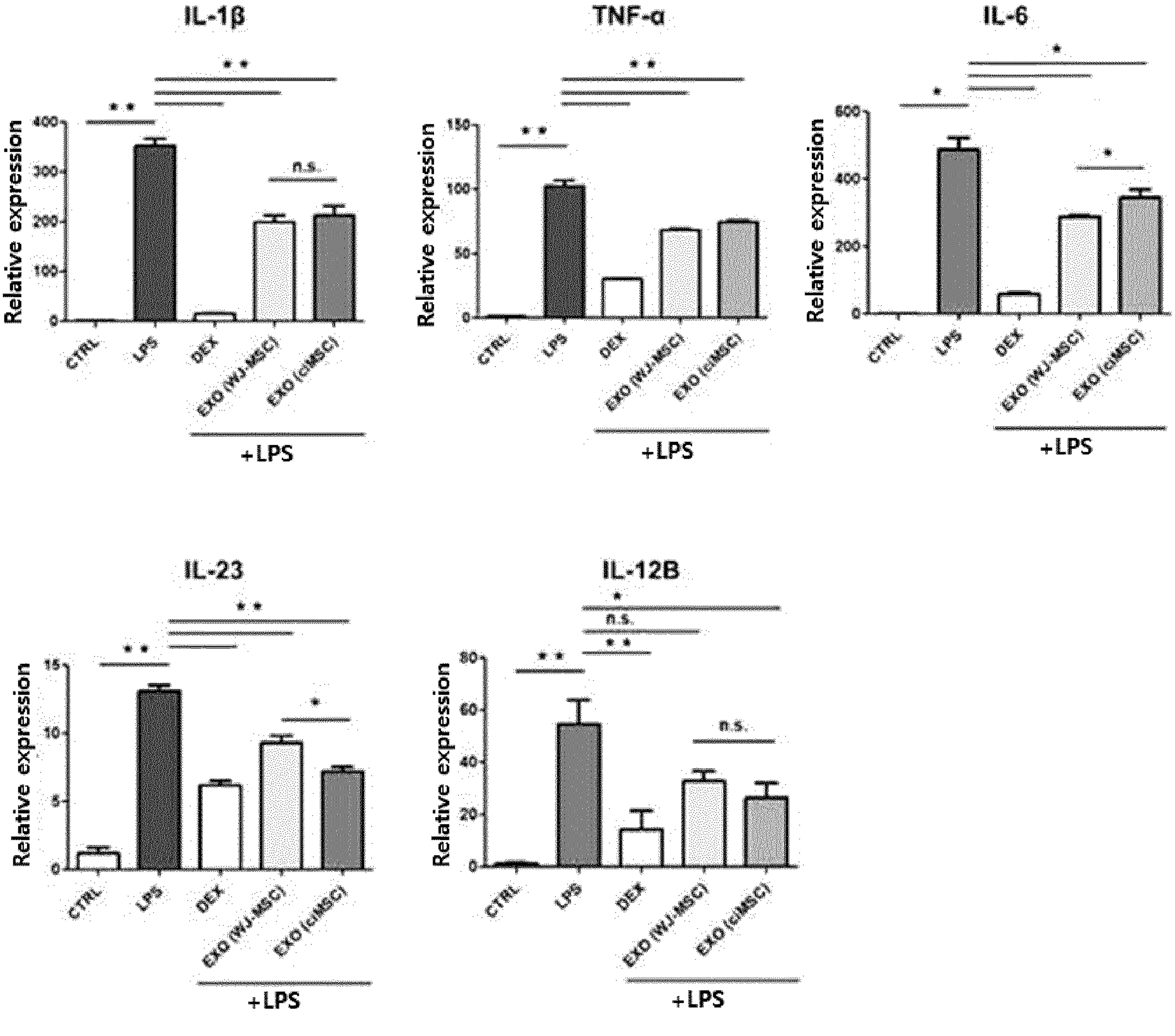
IL-1β
TNF-α
IL-6
IL-23
IL-12B
Relative expression
CTRL
LPS
DEX
EXO (WJ-MSC)
EXO (ciMSC)
+LPS
n.s.

Figure 9

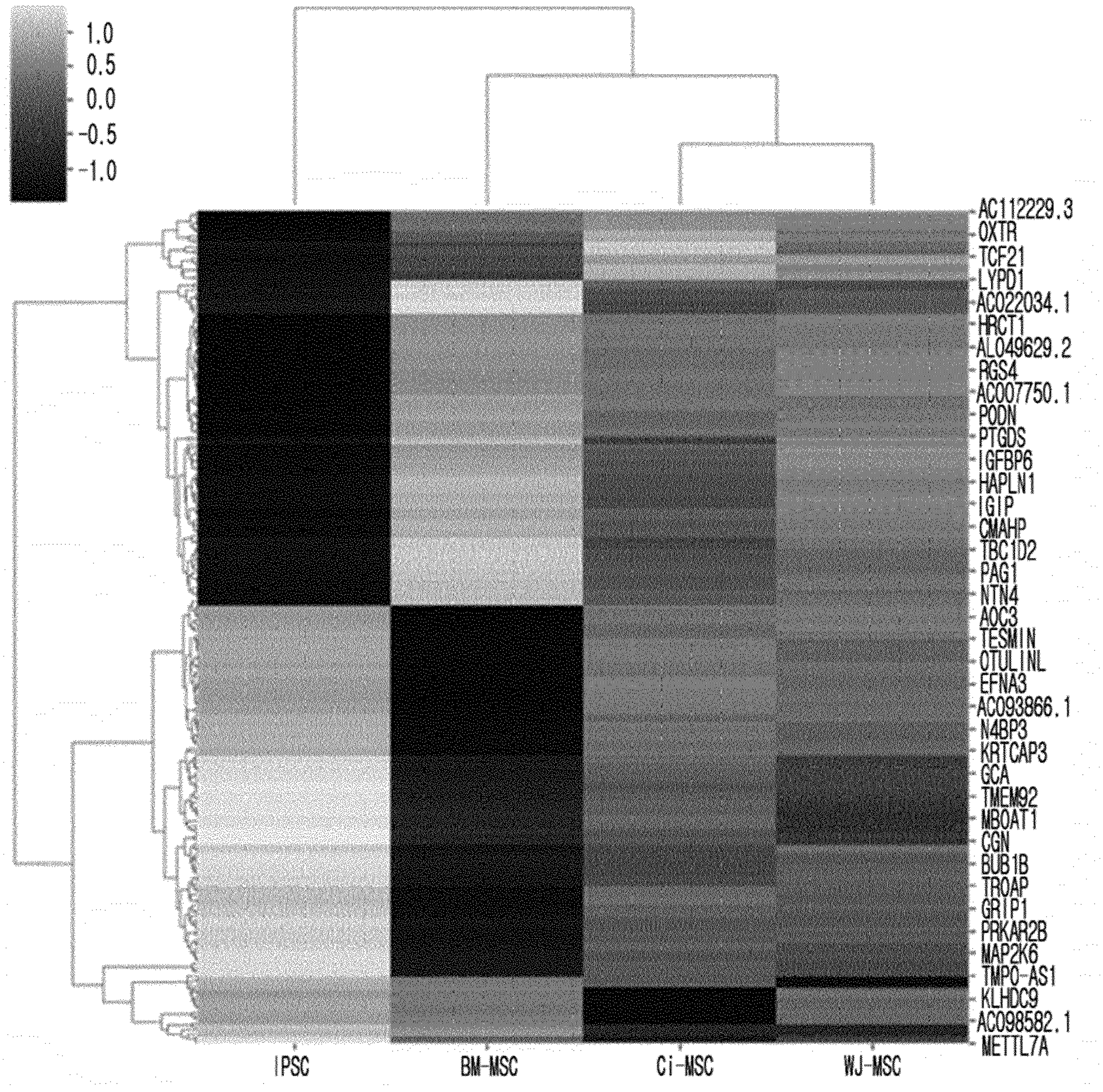
1.0
0.5
0.0
-0.5
-1.0
AC112229.3
OXTR
TCF21
LYPD1
AC022034.1
HRCT1
AL049629.2
RGS4
AC007750.1
PODN
PTGDS
IGFBP6
HAPLN1
IGIP
CMAHP
TBC1D2
PAG1
NTN4
AOC3
TESMIN
OTULINL
EFNA3
AC093866.1
N4BP3
KRTCAP3
GCA
TMEM92
MBOAT1
CGN
BUB1B
TROAP
GRIP1
PRKAR2B
MAP2K6
TMPO-AS1
KLHDC9
AC098582.1
METTL7A
IPSC
BM-MSC
Ci-MSC
WJ-MSC

**INTERNATIONAL SEARCH REPORT**

International application No. **PCT/KR2023/004970**

**A. CLASSIFICATION OF SUBJECT MATTER**

**C12N 5/0775**(2010.01)i; **A61K 35/12**(2006.01)i; **A61P 29/00**(2006.01)i; **A61K 8/98**(2006.01)i

According to International Patent Classification (IPC) or to both national classification and IPC

**B. FIELDS SEARCHED**

Minimum documentation searched (classification system followed by classification symbols)

C12N 5/0775(2010.01); A61K 35/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: BMP4 신호전달 작용제(BMP4 agonist), 유도 만능 줄기 세포(induced pluripotent stem cell), 중간엽 줄기세포(mesenchymal stem cell), 세포외 소포체(extracellular vesicle)

**C. DOCUMENTS CONSIDERED TO BE RELEVANT**

| Category* | Citation of document, with indication, where appropriate, of the relevant passages | Relevant to claim No. |
|---|---|---|
| X<br>Y | CN 113462642 A (CHENGNUO REGENERATIVE MEDICINE TECH ZHUHAI HENGQIN NEW AREA CO., LTD.) 01 October 2021 (2021-10-01)<br>See claims 1 and 2; and paragraphs [0085] and [0163]. | 1,2,5-7<br>3,4,8-11,13 |
| Y | US 2018-0055887 A1 (ACADEMIA SINICA) 01 March 2018 (2018-03-01)<br>See paragraphs [0011] and [0179]-[0181]. | 3,4 |
| Y | KR 10-2319735 B1 (KONKUK UNIVERSITY INDUSTRIAL COOPERATION CORP.) 01 November 2021 (2021-11-01)<br>See claims 1, 2, 12 and 13. | 8-11,13 |
| A | TEHRANI, R. S. H. et al. A comparison between BMP4 and SB4 in inducing germ line gene expression pattern during embryonic stem cells differentiation. Differentiation. electronic publication date 24 November 2021, vol. 123, pp. 9-17.<br>See entire document. | 1-11,13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2023** | **21 July 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/004970**

**C. DOCUMENTS CONSIDERED TO BE RELEVANT**

| Category* | Citation of document, with indication, where appropriate, of the relevant passages | Relevant to claim No. |
|---|---|---|
| A | FENG, L. et al. Discovery of a small-molecule BMP sensitizer for human embryonic stem cell differentiation. Cell reports. 2016, vol. 15, pp. 2063-2075. See entire document. | 1-11,13 |

Form PCT/ISA/210 (second sheet) (July 2022)

**INTERNATIONAL SEARCH REPORT**

International application No.
**PCT/KR2023/004970**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

   Claim 12 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/004970**

| Patent document cited in search report | Publication date (day/month/year) | Patent family member(s) | Publication date (day/month/year) |
|---|---|---|---|
| CN 113462642 A | 01 October 2021 | CN 115094031 A | 23 September 2022 |
| | | WO 2023-016246 A1 | 16 February 2023 |
| US 2018-0055887 A1 | 01 March 2018 | TW 201819625 A | 01 June 2018 |
| KR 10-2319735 B1 | 01 November 2021 | AU 2021-264368 A1 | 01 December 2022 |
| | | CN 115485366 A | 16 December 2022 |
| | | EP 4144836 A1 | 08 March 2023 |
| | | JP 2023-523995 A | 08 June 2023 |
| | | WO 2021-221471 A1 | 04 November 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020190003336

### Non-patent literature cited in the description

- Remington's Pharmaceutical Sciences. 1995